# EUROPEAN PATENT APPLICATION

(11) **EP 1 160 315 A1**
(43) Date of publication of application: **05.12.2001**
(21) Application number: 00810465.5
(22) Date of filing: 29.05.2000
(51) Int. Cl.: C12N 5/00, C12N 5/08

(54) **Use of biochemical factors for tissue engineering**

(71) Applicant: Kantonsspital Basel, 4031 Basel (CH)
(72) Inventor: Martin, Ivan, 4104 Oberwil (CH); Jakob, Marcel, 8586 Andwil (CH); Démarteau, Olivier, 68300 Saint-Louis (FR)
(74) Representative: Schwander, Kuno

(57) **Abstract**

Tissue engineering is the development of biological substitutes to restore, maintain, or improve tissue function. One strategy that has been created to regenerate new tissue comprising the steps of
- providing cells,
- expanding the isolated cells in a first culture medium in which the cells lack differentiated functions and
- redifferentiating the expanded cells in a second cell culture medium.
The present invention provides an improved method for tissue engineering. In particular the method provides specific biochemical factors to supplement cell culture medium during the redifferentiation process with the goal of regenerating tissue equivalents that resemble natural tissues both structurally and functionally. These specific chemical factors induce and/or accelerate and/or promote the redifferentiation of the previously expanded cells. Specifically, the invention provides a method for redifferentiation of mammalian or human chondrocytes in the presence of prostaglandin E2.

## Description

Tissue engineering is the development of biological substitutes to restore, maintain, or improve tissue function. Specifically, tissue engineering is a method by which new living tissues are created in the laboratory to replace diseased or traumatised tissue.

One strategy that has been created to regenerate new tissue is to isolate specific cells from tissue, expand the isolated cells in vitro, and implant the expanded cells into the diseased or traumatised tissue so that the implanted cells proliferate in vivo and eventually replace or repair the tissue defect. This technique has been applied to a variety of cell types and tissue defects. Isolated cells could be either differentiated cells from specific tissues or undifferentiated progenitor cells (stem cells). In both cases, establishment of appropriate culture conditions for cell expansion is extremely important in order to maintain or improve their potential to regenerate structural and functional tissue equivalents.

A particular area of focus for the development of tissue regeneration techniques is the correction of defects in cartilaginous tissue. Unlike other tissues, cartilage has little ability to regenerate itself after trauma, disease or as a result of old age. This is due to the avascular nature of normal articular cartilage. Although damage to the superficial chondral plate generally does not heal, the subchondral bone is vascularised, therefore damage to this location does heal to a limited degree. The new cartilage that grows in place of the damaged, articular cartilage is called fibrocartilage. Fibrocartilage lacks the durability and more desirable mechanical properties of the original hyaline cartilage. People who suffer joint damage are thereafter predisposed to arthritic degeneration.

Several different approaches have been taken to repair cartilage tissue, including chondral shaving, subchondral drilling, and tissue auto/allografts. Other experimental approaches for articular cartilage repair consist in harvesting chondrocytes from a cartilage biopsy and seeding the chondrocytes directly onto a three dimensional transplantation matrix material before implantation of the graft into the damaged area. This technique results in high quality cartilage once regeneration is complete; however, it would require a large quantity of starting material to be harvested from the patient, resulting in increased patient trauma.

In other approaches Chondrocytes are isolated from a biopsy, expanded in monolayer cultures until a sufficient number of cells are obtained and implanted into the damaged area of tissue. Also in these cases, the implantation requires first that the cells are either embedded in a gel or associated with a biodegradable polymer scaffold. The three dimensional nature of those matrices imparts structural integrity to the implant and provides rigid support for growth of the chondrocyte cells into cartilaginous tissue. Although this system has the advantage of requiring fewer cells as starting material, the cartilage obtained by this methods is often of poor quality if the cells are harvested or obtained from skeletally mature donors (adults).

When chondrocytes from cartilage tissue are released from the cartilage matrix and placed in a monolayer culture for expansion until a sufficient number of cells is obtained, they stop producing characteristic markers that define them as being differentiated. Two such markers for differentiated chondrocyte cells are cartilage proteoglycan (aggrecan) and type II collagen. In the expansion environment, chondrocytes proliferate and gradually lose their differentiated phenotype, as indicated by the loss of synthesis of aggrecan and type II collagen and increased production of collagen type I. Dedifferentiation can be prevented or reserved by culturing chondrocytes under conditions that inhibit cell flattening, such as at high cell density, in liquid suspension, in collagen, in agarose and in alginate gels, on substrates with reduced adhesivity, or in the presence of actin disrupting agents. However, mammalian chondrocytes that were dedifferentiated for prolonged periods by serial passaging generally exhibit a reduced potential to redifferentiate within a given time frame, suggesting either a significant decline in the rate of phenotype reversion or a loss of the ability to fully reenter the differentiation program.

Based on this knowledge an other approach has been created to regenerate new cartilage tissue. The corresponding method comprising the steps of
- expanding the isolated cells in a monolayer culture medium in the presence of growth factors in order to maintain their potential to redifferentiate in the expansion environment and
- redifferentiating the expanded cells in a second cell culture medium, as for example in a three-dimensional tissue construct.

In "Mammalian Chondrocytes Expanded in the Presence of Fibroblast Growth Factor 2....", Experimental Cell Research, 253, 681-688, 1999, it is reported that chondrocytes expanded in the presence of fibroblast growth factor-2 (FGF-2) de-differentiate, but maintain their potential for redifferentiation in response to environmental changes. After seeding onto three-dimensional polymer scaffolds, chondrocytes expanded in the presence of FGF-2 form cartilaginous tissue that is histologically and biochemically comparable to that obtain using freshly isolated chondrocytes (primary chondrocytes), in contrast to chondrocytes expanded to the same degree but in the absence of FGF-2. The presence of FGF-2 inhibits the formation of thick F-actin structures, which otherwise is formed during monolayer expansion. This study provides evidence that FGF-2 maintains the chondrogenic potential during chondrocytes expansion in monolayers, possibly due to changes in the architecture of F-Actin elements and allows more efficient utilisation of harvested tissue for cartilage tissue engineering.

Although growth factors can in principal modulate cell proliferation and differentiation by maintaining the chondrogenic potential of the cells during expansion in monolayers, there exists the need for improved redifferentiation techniques for cells that are to be used in tissue engineering. In particular, it has not yet been demonstrated that the expression of collagen type I, a marker of dedifferentiated chondrocytes, can be significantly reduced by the addition of biochemically active factors.

It has now been found, surprisingly, that active agents such as eicosanoids as for example prostaglandines, precursors of eicosanoids, as for example arachidonic acid, and mediators of wound healing acting in concert with eicosanoids, as for example histamine and dexamethasone, are highly efficacious as biochemical factors for cell culturing, specifically for cell-differentiation, for example for the acquisition of the chondrocyte-specific phenotype.

The stated object is therefore achieved by the use of the biochemical factors according to claim 1 in tissue engineering.

An other object of the invention is the use of prostaglandines during the expansion phase of a cell population in a manner that results in successful proliferation of the cells and maintenance of their differentiation potential

It is a further object of the invention to provide a method for culturing tissues, namely a method according to claim 4 for use in tissue engineering.

Advantageous embodiments of the invention become evident from the dependent claims.

The method according to the invention comprises the steps of:
- providing a cell population;
- expanding the isolated cells in a monolayer culture medium in which the cells lack differentiated functions and
- redifferentiating the expanded cells in a second cell culture medium in the presence of at least one biochemical factor which induces and/or accelerates and/or promotes the redifferentiation of the cells.

Any of variety of biochemical factors that increase differentiation of the cells can be used in the process of cell redifferentiation according to claim 4. Non-limiting examples of biochemical factors that may be used in the present invention's method are arachidonic acid, prostaglandin A, prostaglandin B, prostaglandin E, prostaglandin F, and histamine, with or without additional hormones/corticoids, like dexamethasone, and growth factors, like TGFβ.

The biochemical factors are preferably able to induce and promote redifferentiation of the cells which has been previously isolated from mature tissue and dedifferentiated under expansion conditions. According to the present invention, a biochemical factor that, when added to tissue culture medium during redifferentiation, decreases the collagen type I production of that cell population is preferred.

The present invention demonstrates that the biochemical factors mentioned above promote the redifferentiation process of chondrocytes isolated from mature cartilage tissue and expanded in monolayer culture following transfer of the cells into a differentiation environment. Specifically, after two weeks of culturing expanded cells in a redifferentiating environment the differentiation indexes were higher in all culture conditions where the medium was supplemented with prostaglandin E2 compared to chondrocytes redifferentiated in the absence of prostaglandin E2. According to the invention it is defined the ratio of mRNA levels of collagen type II to I as differentiation index, since collagen type II is a typical marker of differentiated chondrocytes, as opposed to collagen type I, which is expressed by dedifferentiated chondrocytes.

A variety of cell types can be used in the present invention. According to the present invention, progenitor (stem) cells can be used to generate new tissue, and any cell type that can be isolated and expanded is usable to regenerate new tissue. Non-limiting examples include endothelial cells, muscle cells, chondrocytes and melanocytes.

In a preferred embodiment of the invention according to claim 4, chondrocytes, preferably mammalian, and more preferably human, are
- isolated from mature cartilage tissue,
- expanded in vitro in monolayer culture medium and
- transferred for redifferentiation into a second culture medium containing prostaglandin E2 and/or arachidonic acid and/or dexamethasone.

In another preferred embodiment of the invention according to claim 4, redifferentiation is performed preferably in a serum-free medium. More preferably, redifferentiation is performed in a serum-free medium containing prostaglandin E2 and dexamethasone. Most preferably, redifferentiation is performed in a serum-free medium containing prostaglandin E2, arachidonic acid, histamine and dexamethasone.

The condition of the expanded cells significantly affects the successful regeneration of quality tissue. Therefore, it is preferable that the expanded cells are homogeneous with respect to their stage of differentiation and that therefore the growth environment is manipulated by the addition of growth factors and/or hormones to achieve a homogeneous population of dedifferentiated cells and to increase the proliferation rate of the chondrocytes while preserving the appropriate differentiation properties of the cells so that a successful regeneration of high quality cartilage tissue can be ensured for implantation. Examples of growth factors that can be used are: platelet derived growth factors, epidermal growth factors, heparin binding factor, transforming growth factor alpha and beta, alpha fibroblastic growth factor, fibroblast growth factor 2 (FGF-2), insulin like growth factors, bone morphogenetic proteins, and vascular endothelium growth factor. Examples of hormones that can be used are the prostaglandines, as for example prostaglandine E2.

In another preferred embodiment of the invention according to claim 4, mammalian chondrocytes, preferably human, are expanded in a medium containing FGF-2, platelet derived growth factor and transforming growth factor beta (TGFβ).

Human chondrocytes expanded in a cell culture medium containing FGF-2 are preferentially redifferentiated in a cell culture medium which is substantially free of serum and contains insulin, transferrin, selenous acid, linoleic acid, bovine serum albumin and at least one of the biochemical factors mentioned above.

In another embodiment of the invention hormones (e.g., insulin glucagon or estrogen) and/or angiogenic factors may be used for in vitro proliferation, i.e. expansion. Chondrocytes freshly isolated from cartilaginous tissue are normally responsive to insulin which causes increased proliferation of the chondrocytes. Chondrocytes first expanded in the presence of FGF-2 are responsive to insulin in a manner similar to chondrocytes harvested directly from cartilage tissue and seeded directly onto the implantation matrix without an intervening expansion step. Since FGF-2 expanded chondrocytes are highly responsive to insulin in a similar fashion as freshly harvested chondrocytes, they might represent an appropriate cell population for cartilage regeneration in those therapies involving the use of additional hormones and growth factors to further stimulate tissue regeneration.

Those of ordinary skill in the art will appreciate the variety of cell types to which the inventive method of cell expansion and redifferentiation can be applied. Tissue engineering techniques have been used to correct defects by using a myriad of different cell types. Tissue engineering can be applied to the correct on of hard tissue defects, such as defects in cartilage or bone that arise from disease or trauma. Tissue engineering has also been applied to the correction of soft tissue structures. By way of example, cells used in the current invention can be used to regenerate metabolic organs (the liver or pancreas) epidermal tissue (e.g. tissue of burn victims) or to reconstruct or augment breast tissue (e.g. muscle cells may be used to reconstruct the breast of women afflicted with breast cancer, congenital defects, or damage resulting from trauma; see U.S. Patent No. 5,512,600 and WO/96/18424, both of which are incorporated herein by reference). Furthermore, congenital defects such as vesicoureteral reflux, or incontinence can be corrected by implantation of a gel or scaffolding matrix seeded with muscle cells in an effective amount to yield a muscle area that provides the required control over the passage of urine or otherwise corrects the defect (U.S. Patent No. 5,667,778, incorporated herein by reference).

According to the present invention, the cells used to reconstruct or augment the specific physical location can be different from the cells that normally constitute that tissue in the body. For example, chondrocytes can be used to correct soft tissue defects by serving as bulking agent.

It is known that mammalian cells (e.g., chondrocytes and bone) in a three dimensional environment respond very differently to stimuli (e.g., biochemical factors and hydrodymanic factors or signals) than do cells in monolayer cultures. It has been demonstrated that the differentiated phenotype of chondrocyte cells can be stabilised by transferring them for redifferentiation from a monolayer culture into a three dimensional environment, as for example by seeding onto biodegradable polymer scaffolds (e.g., meshes made of a poly-glycolic acid) or by forming spherical pellets in conical tubes.

Preferably the cells are autologous cells. Alternatively the cells are isolated from a close relative or from an individual of the same species. It will be appreciated by those of ordinary skill in the art that a cell population that is responsive to proliferation or differentiation cell stimuli will be advantageous for use in tissue engineering. A cell population that can respond better to such stimuli will regenerate more quickly, more dependably and as a result yield a higher quality tissue for implantation.

In yet another embodiment of the invention, expansion of cells also improves the efficiency of transfection of nucleic acids into the cells. Typically, gene transfer is carried out during monolayer expansion. Therefore, applications where tissue engineering techniques are combined with gene therapy may be utilised in accordance with the teachings of the present invention. For example, cells may be transfected with a vector which confers resistance to a variety of biological and chemical compounds as for example to antibiotics, cytokines and inflammatory agents.

Cells redifferentiated according to the invention can be implanted with suitable biodegradable, polymeric matrix to form new tissue. There are different forms of matrices which can be used. Non-limiting examples include a polymeric gels formed of a material such as alginate having cells suspended therein, fibrous matrices having an interstitial spacing between about 100 and 300 µm, and 3D foams. Matrices can be based on naturally occurring polymers (e.g., hyaluronic acid, collagen, etc.) or synthetic polymers(e.g., poly-glycolic acid, poly-lactic acid, etc.), or both. For a detailed description of hydrogel polymer solutions and polymeric matrices, and other methods of implantation see US Patent 5,716,404. For other methods of using biodegradable polymers to regenerate metabolic organs and other tissues, see Cima et al., Biotechn. Bioeng., 38, 145-158, 1991; Langer al., Biomaterials, 11, 738-745, 1990; Vacanti et al., J. Pediatr. Surg., 23, 3-9,1988; and Vacanti et al., Arch. Surg., 123, 545-549, 1988.

In some embodiments, the cell-matrix structures are implanted in combination with tissue expander devices. As the cell-matrix is implanted, or cells proliferate and form new tissue, the expander size is decreased, until it can be removed and the desired reconstruction or augmentation is obtained.

The present invention will now be illustrated in more detail by the following examples, which are not meant to limit the scope of the invention. These examples are described with reference to the drawing. In the drawing,

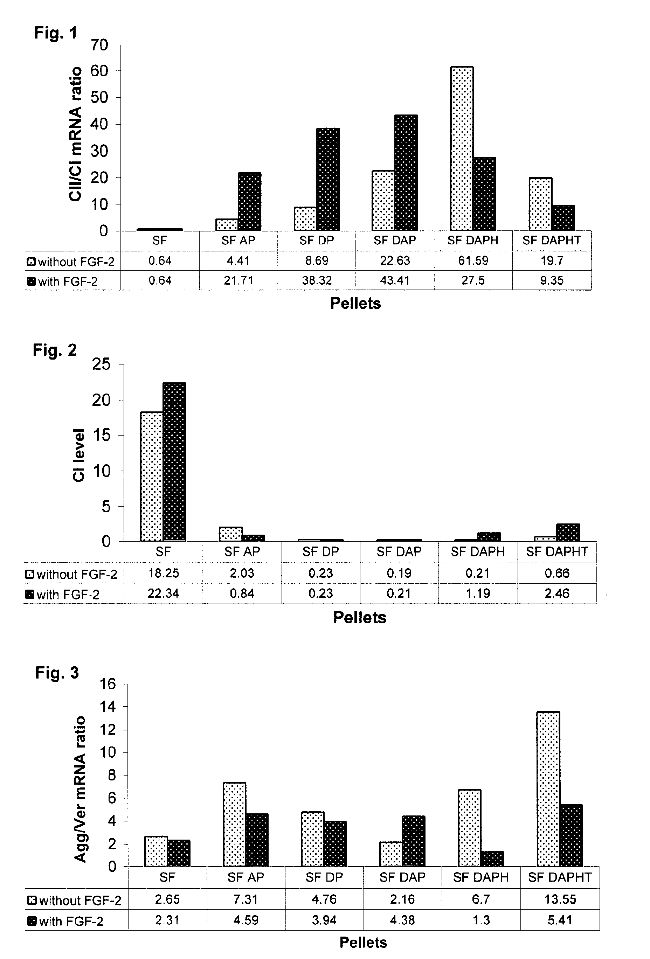

Figure 1 shows a graph representing the differentiation indexes CII/CI of six pellets based on human chondrocytes redifferentiated in different culture mediums and previously expanded with or without FGF-2,
Figure 2 shows a graph representing the levels of type I collagen mRNA of the six pellets mentioned above,
Figure 3 shows a graph representing the differentiation indexes Agg/Ver ratio of the said six pellets, and
Figure 4 is a safranin O-stained histological section of a human chondrocyte-PGA mesh construct cultured for 6 weeks in the presence of dexamethasone, arachidonic acid, prostaglandin E2, histamine and TGFb. Human chondrocytes have been previously expanded with PDGFbb, FGF-2 and TGFβ.

### Cultivation Phase I: Cell isolation and expansion in monolayers:

Human articular cartilage samples were collected from the hip of 3 patients (67, 73, and 84 years old) with no history of joint disease, undergoing joint replacement, following femoral neck fracture.

The cartilage samples were harvested aseptically and digested with 0,15% type II collagenase for 22 hours to isolate primary chondrocytes. The cells were washed and resuspended in Dulbecco's modified Eagles medium (DMEM) supplemented with 10% fetal bovine serum, 4'500 mg/l D-Glucose, nonessential amino acids, 1mM sodium pyruvate, 100 mM HEPES buffer, 100 U/ml penicillin, 100 µg/ml streptomycin, and 0,29 mg/ml L-glutamine (control medium, CTR). Chondrocytes were seeded into tissue culture flasks at approximately 10⁴ cells/cm² and cultured in a humidified 37°C / 5% CO² incubator.

After approximately 10 days, when the cells were confluent, first passage cells (P1) were detached using 0,25% trypsin/1 mM EDTA and replated at 5 x 10³ cells/cm². After one more week, when the cells reached again confluency, second passage (P2) cells were trypsinised and cultured in pellets. Throughout the expansion phase, cells were cultured in control medium, with or without the addition of 5 ng/ml of Fibroblast Growth Factor-2 (FGF-2).

### Phase II: Cultivation of human chondrocytes in pellets:

The cells were suspended at 5 x 10⁵ cells/0,5 ml of serum-free medium (SF) consisting of DMEM supplemented with ITS+1 (containing 10 µg/ml insulin, 5,5 µg/ml transferrin, 5 ng/ml selenium, 0,5 mg/ml bovine serum albumin, 4,7 µg/ml linoleic acid), 0,1 mM ascorbic-acid 2-Phosphate, and 1,25 µg/ml human serum albumin. This suspension was centrifuged at less than 8000 rpm for 15 seconds in polypropylene conical tubes to form spherical pellets which were then statically incubated in a humidified 37°C / 5% CO² incubator for 24 hours. The pellets were then placed onto an orbital shaker (30 rpm) and were cultured for 2 to 4 weeks in SF supplemented with different combinations of
- 10⁻⁷ M dexamethasone(D),
- 1 µg/ml arachidonic acid (A),
- 1 µg/ml prostaglandin E2 (P), and
- 10⁻⁴ M histamine (H).

P, alone or in combination with A and/or D and/or H and/or T, was supplemented to the SF medium during 3D culture of the human chondrocyte cell pellets. After two weeks, the differentiation indexes (CII/CI and Agg/Ver) were assessed.

The following six experimental groups were formed:
- SF
- SF+A+P
- SF+D+P
- SF+D+A+P
- SF+D+A+P+H
- SF+D+A+P+H+T (TGFβ)

Further, for each primary culture, 2 pellets were formed per experimental group and assessed histologically and for quantitative PCR as described below.

### Histology:

The pellets were fixed in 4% buffered formalin for 24 hours at 4°C. They were then embedded in paraffin, and cut into 5 µm sections. Samples slices were stained with Safranin O for sulfated glycosaminoglycans (GAG).

### Real-Time quantitative PCR:

*Theoretical Basis.* Real-time quantitative PCR monitors the degradation of a sequence-specific, dual-labeled fluorescent probe after each cycle of PCR amplification. During the extension phase, the 5'-exonuclease activity of Taq DNA polymerase cleaves the probe, separating the 5'-reporter fluorescent dye from the 3'-quencher fluorescent dye, resulting in an increase in the emission spectra of the reporter fluorescent dye. After subtraction of the background fluorescence, calculated during the first 15 amplification cycles, the measured fluorescence is graphed as an amplification plot. Each reaction is characterised by a value, Ct, defined as the fractional number of cycles at which the reporter fluorescent emission reaches a fixed threshold level in the exponential region of the amplification plot. The Ct value is correlated to input target mRNA amount: a larger starting quantity of mRNA target results in a lower number of PCR cycles required for the reporter fluorescent emission to reach the threshold, and therefore a lower Ct value. Thus, the method is not based on the measurement of the total amount of amplified product after a fixed number of cycles, as in conventional PCR, and does not require post-PCR processing of the product (Gibson UE et al., 1996).

*Primers and Probes.* Primers and probes for human GAPDH, Collagen types I, II, Aggrecan, and Versican were designed with the assistance of the Primer Express computer program (Perkin-Elmer Applied Biosystems, Foster City, CA), in order to display minimal internal structure (i.e., primer-dimer formation) and similar melting temperatures. The total gene specificity of the nucleotide sequences chosen for the primers and probes was confirmed by BLASTN searches (GenBank database sequences). To avoid non-specific fluorescent emission derived from the recognition of contaminating genomic DNA by the probe, the middle third of the probe was placed at the junction between two exons. Primers were purchased from Microsynth (Balgach, Switzerland) and probes were from Perkin-Elmer Applied Biosystems or Eurogentech (Seraing, Belgium). Optimal concentrations for the designed primers and probes were determined as the lowest ones giving the highest fluorescence levels and the lowest Ct values. The efficiency of the amplification for each target gene, assessed as described in (Jakob et al.) was always higher than 90 %.

*Total RNA extraction and cDNA synthesis.* RNA was extracted from pellets using the standard single-step acid-phenol guanidinium method (Chomczynski P et al., 1987). cDNA was generated from 2 µg of RNA by using murine MLV reverse transcriptase (BRL, Gaithersville, MD) in the presence of dNTPs and DTT, according to the manufacturers' instructions.

*PCR Amplification and Analysis.* PCR reactions were performed and monitored using a ABI Prism 7700 Sequence Detection System (Perkin-Elmer Applied Biosystems). The PCR master mix was based on AmpliTaq Gold DNA polymerase (Perkin-Elmer Applied Biosystems). cDNA samples (2,5 µl in a total of 25 µl per well) were analysed in single or in duplicate. Primers and probes were used at concentrations ranging from 50 to 900 nM. After an initial denaturation step at 95°C for 10 min., the cDNA products were amplified with 50 PCR cycles, consisting of a denaturation step at 95°C for 15 s and an extension step at 60°C for 1 min. Data analysis was carried out by using the Sequence Detector V program (Perkin-Elmer Applied Biosystems). For each sample, the Ct value was determined as the cycle number at which the fluorescence intensity reached 0,05; this value was chosen after confirming that in this range all curves were in the exponential phase of amplification. For each cDNA sample, the Ct value of each target sequence was subtracted to the Ct value of the reference gene (GAPDH), to derive ΔCt. The level of expression of each target gene was then calculated as 2^{ΔCt}. This formula is based on the assumption that the efficiencies of amplification for the gene of interest and the housekeeping gene are comparable (< 10% difference) and close to 100% (PE-ABI; Sequence Detector User Bulletin 2). GAPDH was chosen as the reference housekeeping gene based on the majority of previous studies on chondrocyte gene expression.

Since collagen type II and aggrecan are the typical markers of differentiated chondrocytes in hyaline cartilage, as opposed to collagen type I and versican, which are expressed by de-differentiated chondrocytes and in fibrocartilage, the ratios of mRNA levels of collagen type II to I (CII/CI) and of aggrecan to versican (Agg/Ver) defines "differentiation indexes" related to the expression of collagens and proteoglycans, respectively.

### Results:

The redifferentiation of expanded chondrocytes in pellet cultures previously expanded without FGF-2 showed that, CII/CI and Agg/Ver ratios were much higher if pellets were cultured in defined medium containing at least prostaglandin as biochemical factor, compared to the control medium SF. The highest CII/CI mRNA ratio has been detected in the pellet based on SF+D+A+P+H and the highest Agg/Ver mRNA ratio has been detected in the pellet based on SF+D+A+P+H+T.

From the assay it can be concluded, that in all culture conditions where SF medium was supplemented with P, the differentiation indexes (CII/CI and Agg/Ver) were at least 2 orders of magnitude higher compared to the control culture. The strong effect of P on the CII/CI ratio increase was due to a decrease in the expression of CI mRNA.

All these effects were comparable on chondrocytes expanded with FGF-2, suggesting that medium supplementation with P has a beneficial effect also on growth factor-expanded chondrocytes.

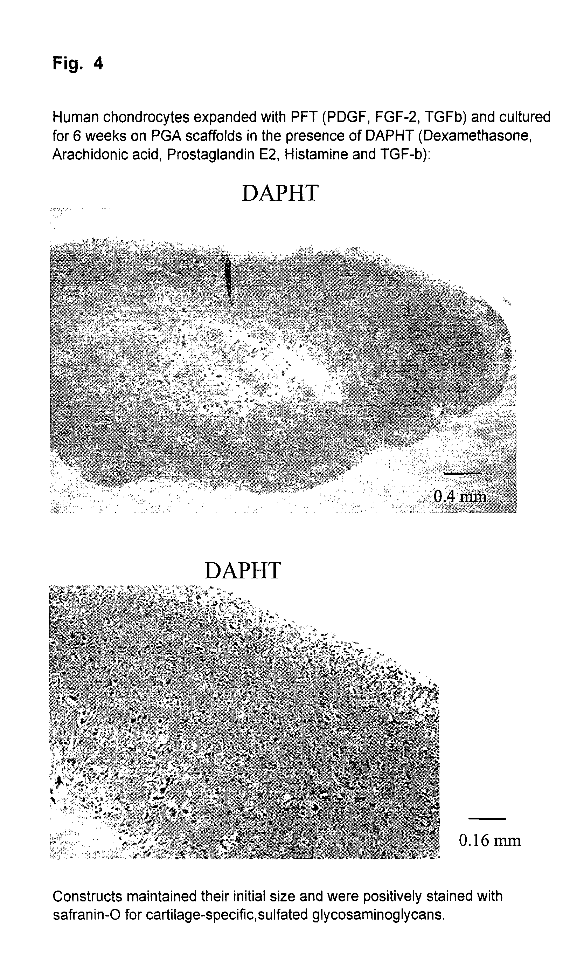

PGA meshes seeded with PDGFbb, FGF-2 and TGFb-expanded chondrocytes and cultured in SF DAPHT medium histologically resembled cartilage tissue, with cells displaying a typical chondrocyte morphology and the surrounding extracellular matrix containing sulfated glycosaminoglycans, as assessed by Hematoxilin/Safranin O stain and shown in figure 4.

## Claims

1. Use of at least one biochemical factor in tissue engineering for the differentiation of progenitor cells and/or the regeneration of the tissue-specific phenotype after cell-dedifferentiation **characterised in that** the at least one biochemical factor is selected from the group consisting of: eicosanoids, as for example prostaglandines, precursors of eicosanoids, as for example arachidonic acid, and mediators of wound healing acting in concert with eicosanoids, as for example histamine and dexamethasone.

2. Use of protaglandines as biochemical factors to support the expansion of cells while maintaining their commitment to differentiate.

3. Use according to claim 1 or 2, wherein the at least one biochemical factor is prostaglandin E2.

4. A method for regenerating tissue comprising the steps of
- providing cells,
- expanding the isolated cells in a first culture medium in which the cells lack differentiated functions and
- redifferentiating the expanded cells in a second cell culture medium,
**characterised in that** the redifferentiation of the expanded cells occurs in the presence of at least one biochemical factor which induces and/or accelerates and/or promotes the redifferentiation of the cells.

5. A method according to claim 4, **characterised in that** the step of providing cells comprises providing mammalian cells.

6. A method according to claim 4, **characterised in that** the step of providing cells comprises providing human cells.

7. A method according to claim 5 or 6, **characterised in that** the step of providing cells comprises providing chondrocytes.

8. A method according to claim 5 or 6, **characterised in that** the step of providing cells comprises providing progenitor cells.

9. A method according to any of claims 4 to 8, **characterised in that** the redifferentiation of the expanded cells occurs in the presence of at least one biochemical factor selected from the group consisting of: eicosanoids as for example prostaglandines, precursors of eicosanoids, as for example arachidonic acid, and mediators of wound healing acting in concert with eicosanoids, as for example histamine and dexamethasone..

10. A method according to claim 9, **characterised in that** the redifferentiation occurs in the presence of prostaglandin E2.

11. A method according to any of claims 4 to 10, **characterised by** the following steps:
- isolation of chondrocytes from mature cartilage tissue,
- expansion of the cells in vitro in monolayer culture medium and
- transferring the expanded cells for redifferentiation into a second culture medium containing a least one of said biochemical factors.

12. A method according to any of claims 4 to 11, **characterised in that** the redifferentiation is performed in a serum-free medium.

13. A method according to claim 12, **characterised in that** the serum free medium containing insulin, transferrin, selenous acid, albumin, linoleic acid and ascorbic-acid.

14. A method according to claim 12 or 13, **characterised in that** the serum-free cell culture medium containing at least one growth factor.

15. A method according to claim 14, **characterised in that** the serum-free cell culture medium containing transforming growth factor beta.

16. A method according to any of claims 4 to 15, **characterised in that** the expansion of the cells is performed in the presence of at least one growth factor.

17. A method according to claim 16, **characterised in that** the step of expanding cells containing at least one factor selected from the group consisting of: platelet derived growth factors, epidermal growth factors , heparin binding factor, transforming growth factor alpha and beta, alpha fibroblastic growth factor, fibroblast growth factor 2, insulin like growth factors, bone morphogenetic proteins, vascular endothelium growth factor, and prostaglandines.

18. A method according to claim 17, **characterised in that** the expansion of the cells is performed in the presence of platelet derived growth factor, transforming growth factor beta and fibroblast growth factor 2.
